# DEMANDE DE BREVET EUROPEEN

(11) **EP 2 404 619 A1**
(43) Date de publication de la demande: **11.01.2012**
(21) Numéro de dépôt: 11170986.1
(22) Date de dépôt: 22.06.2011
(51) Int. Cl.: A61K 49/00, A61K 9/06, A61K 47/36, A61K 6/00, A61K 31/167, A61K 9/00

(54) **Composition injectable comprenant un médicament injectable et un gel**

(30) Priorité: 05.07.2010 FR 1055426
(71) Demandeur: Villette, Alain, 79700 Saint Pierre Les Echaubrognes (FR)
(72) Inventeur: Villette, Alain, 79700 Saint Pierre Les Echaubrognes (FR)
(74) Mandataire: Thinat, Michel

(57) **Abrégé**

L'invention concerne une composition d'un médicament injectable et d'un gel dans le but de concentrer le produit sur place pour renforcer son action et/ou diminuer sa toxicité générale.

## Description

La majorité des médicaments injectables se présentent sous forme de liquides dont la fluidité est équivalente à celle de l'eau. Certains ont une viscosité huileuse. En outre, la plupart des médicaments injectables ont une activité ambivalente, à savoir, qu'ils présentent d'une part, une activité positive curative, c'est la raison de leur utilisation, d'autre part une action négative toxique plus ou moins importante, qui limite leur utilisation. Cette ambivalence intéresse l'ensemble des médicaments injectables.

La présente invention concerne particulièrement les médicaments ayant une action locale. Ces médicaments ont une toxicité locale et aussi générale, ce qui a pour conséquence que certains médicaments, ayant une toxicité « non maîtrisée » ne sont pas utilisés localement.

Tout produit liquide injecté dans l'organisme se dilue dans les liquides biologiques vasculaires, extracellulaires et cellulaires. Cette dilution est néfaste lorsque l'on souhaite une action locale du produit injecté puisqu'elle diminue localement la concentration du principe actif.

L'utilisateur du médicament est bien souvent confronté aux problèmes suivants : augmenter la quantité injectée pour obtenir une activité plus importante mais qui va aller inéluctablement avec une augmentation de la toxicité dont la limite peut être atteinte assez rapidement.

Le problème posé est donc : comment augmenter la puissance d'un médicament sans augmenter sa toxicité ?

Ce problème se pose particulièrement en anesthésie dentaire où, pour obtenir le résultat escompté, on adjoint au principe actif, qui est la molécule anesthésique, un vasoactif, en général de l'adrénaline.

L'adrénaline est une cathécolamine endogène, donc sécrétée en permanence par l'organisme humain.

Ce vasoactif, adjoint à un liquide anesthésiant, entraîne une vasoconstriction des vaisseaux dans la zone injectée.

Cette vasoconstriction diminue le volume des vaisseaux, donc le volume sanguin ; la dilution se voit alors diminuée, ce qui augmente la concentration en principe actif.

En ralentissant sa diffusion dans l'organisme, elle diminue la toxicité. Donc : la présence du vasoactif renforce et prolonge l'action de l'anesthésique et en même temps diminue la toxicité de l'injection.

L'action locale de l'adrénaline peut être néfaste si son action se prolonge.

L'adrénaline, injectée dans un tissu peu irrigué, ou en quantité trop importante, peut entraîner une nécrose du tissu.

Sur le plan général on lui reproche son action hypotensive d'abord et hypertensive ensuite qui peut donner une sensation de malaise (jambes molles) et tachycardisante, mal perçue par les patients et les praticiens.

Les praticiens craignent aussi des effets nocifs sur le coeur.

De même, lui sont reprochés les problèmes (particulièrement l'allergie) posés par les conservateurs et antioxydants adjoints à l'adrénaline pour en assurer sa conservation.

Les effets négatifs de l'adrénaline ou de la noradrénaline qui sont les principaux vasoactifs utilisés, sont décrits dans les documents suivants :
-- Mitsuhiro Haraguchi US 2006/018 9572 Al,
-- Mitsuhiro Haraguchi US 2006/021 6245 Al,
- Al Reader US 6,075,059 A,
-- Mitsuhiro Haraguchi US 6,008,256 A, et
-- Kim K.Forrest US 4,963,345.

La lecture de ces documents montre que leurs auteurs ont pour première préoccupation de remplacer partiellement ou de supprimer totalement les cathécolamines dans les solutions anesthésiques dentaires.

La diminution de la diffusion de la solution injectée peut être obtenue en augmentant la viscosité de la solution injectée. Cette diminution de la diffusion sera fonction de la viscosité de la solution. Plus la solution à une viscosité élevée plus sa diffusion sera limitée. On pourra donc remplacer, avantageusement, l'adrénaline par un gel.

Aussi, la présente invention propose de combiner ou assembler un anesthésique avec un gel, quels que soient les pourcentages relatifs des deux composants et quelles qu'en soient les applications envisagées.

L'adrénaline agit par voie chimique, le gel agit de façon mécanique.

Le gel, en plus de son action mécanique, doit répondre à certains critères :
-- il doit être biocompatible et apyrogène,
-- il ne doit pas générer de douleur lors de l'injection, quelle que soit la densité du tissu,
-- il ne doit pas empêcher le passage du produit à travers la corticale osseuse,
-- il doit être totalement résorbable.

Selon la viscosité de la solution, celle-ci diffusera moins loin à partir du point d'injection comparée à une solution aqueuse.

La sensation d'engourdissement des tissus mous, désagréablement perçue par les patients, sera limitée.

Le gel incorporé à la solution anesthésique est, dans la présente invention, un gel résorbable de hyaluronidate de sodium, d'origine animale ou végétale, réticulé ou non, ou tout autre gel bio compatible.

Dans son brevet US 2006/018 9572 Al Mitsuhiro Haraguchi revendique l'utilisation conjointe de chondroitine sulfate et d'hydroxypropyl méthylcellulose associée à de la lidocaïne.

Selon la présente invention, il n'est utilisé qu'un seul type de gélifiant.

L'acide hyaluronique à partir duquel est réalisé le hyaluronidate de sodium est un acide mucopolysaccharide comme le chondroitine sulfate.

C'est le gel utilisé dans les études cliniques réalisées.

Les tests cliniques ont consisté à élaborer des solutions de lidocaïne, articaïne et mépivacaïne et prilocaïne contenant des concentrations en molécule anesthésique identiques aux solutions utilisées actuellement, soit 2 % de lidocaïne, 4 % d'articaïne, 3 % de mépivacaïne et 4 % de prilocaïne.

À chaque solution a été ajouté du hyaluronidate de sodium en quantités diverses pour obtenir des viscosités différentes permettant de réaliser des injections indolores.

L'adjuvant utilisé pour compléter le mélange à concurrence d'1,8 ml (volume d'une cartouche anesthésique) et du chlorure de sodium en solution isotonique. On aurait pu, tout aussi bien, utiliser un sel de potassium.

On obtient trois viscosités, chacune adaptée à une technique anesthésique.

Les viscosités obtenues sont mesurées avec un rhéomètre a contrainte imposée plus géométrie plan sur plan. Les valeurs indiquées correspondent à une viscosité dynamique et est exprimée en Pascal par seconde, (PA/s).

Les valeurs indiquées ci-dessous ne sont pas restrictives, elles ne sont citées qu'à titre d'exemples pour montrer que l'on a essentiellement trois grandes familles de produits qui se distinguent par leur viscosité dont l'ordre de grandeur est indiquée ci-après:
**Viscosité 1 :** 17,5 PA/s, pH 6,2, est obtenue en assemblant 1 ml de hyaluronidate de sodium plus 0,8 ml d'articaïne à 9 %. Cette viscosité est destinée aux tronculaires et aux tissus mous.
**Viscosité II :** 8,3 PA/s, pH 5,75, obtenue en assemblant 0,9 ml de hyaluronidate de sodium plus 0,8 ml d'articaïne plus 0, 1 millilitre de chlorure de sodium. Cette viscosité est destinée aux paraapicales et éventuellement aux anesthésies diploïques dans un os peu dense.
**Viscosité III :** 3,05 pH, 5, 27, PA/S obtenue en assemblant 0, 8 ml de hyaluronidate de sodium plus 0, 8 ml d'articaïne plus 0, 2 ml de chlorure de sodium. Cette viscosité est destinée aux anesthésies diploïques et intraseptales.

Ces assemblages peuvent être déclinés avec de la lidocaïne, de la mépivacaïne et de la prilocaïne exactement dans les mêmes proportions.

Ces assemblages peuvent être déclinés, pour le gel soit en version normale soit en version réticulée. La version réticulée prolonge l'action de la molécule anesthésiante.

Les pH obtenus sont globalement supérieurs de 0,7 à ceux des solutions classiques, ce qui améliore la toxicité cytologique de ces nouvelles solutions.

Une autre famille de solutions a été élaborée en assemblant les solutions présentées ci-dessus en y adjoignant de l'adrénaline à raison de 0,0050 mg par millilitre pour obtenir une solution dosée à 1/ 200 000e et 0,01 mg par millilitre pour obtenir une solution à 1/ 100 000e.

Ces solutions sont destinées à être utilisées en chirurgie étendue, pour diminuer le saignement.

En quelque sorte une petite action vasoconstrictive potentialise l'action mécanique du gel.

Cette addition de vasoactif peut se faire avec toutes les molécules anesthésiques existantes.

### Tests cliniques

Ces solutions ont été comparées, à quantité égale, aux solutions classiques, sans vasoconstricteur (mépivacaïne, lidocaïne, articaïne) et à 1/200 000e et 1/100 000e d'adrénaline dans les différentes techniques anesthésiques (paraapicales, tronculaires, intraligamentaires, ostéocentrale et transcorticale).

Pour l'ensemble des techniques anesthésiques, la durée d'anesthésie obtenue avec les solutions gélifiées est le double de la durée obtenue avec les solutions sans vasoactif, et l'équivalent des solutions àl/200 etl/100 000e d'adrénaline.

Les études comparatives ont montré qu'on pouvait obtenir exactement le même effetmême efficacité, même durée -- en remplaçant l'adrénaline par un gel.

Les résultats obtenus en anesthésie dentaire, à savoir : conservation d'un effet anesthésique équivalent aux plus puissantes solutions anesthésiques actuelles en ayant remplacé l'intégralité des cathécolamines par du gel résorbable peuvent être transposés à d'autres médicaments injectables.

L'adjonction d'un gel à un médicament injectable, à action locale, permet de limiter la diffusion de celui-ci, donc d'augmenter sa concentration sur place et de diminuer sa toxicité générale.

Ce principe peut est appliqué dans de nombreux domaines, là où l'action d'un produit doit être ciblée et où la toxicité générale, par diffusion, doit être limitée.

Exemple : traitement d'une tumeur par l'injection d'un produit gélifié, en vue de la scléroser ou de la détruire.

Dans ce genre de traitement, on peut additionner les deux effets, en adjoignant au produit gélifiant un vasoconstricteur qui limitera encore plus la diffusion du produit.

La réticulation plus ou moins importante du gel entraîne une résorbabilité plus ou moins prolongée.

La résorbabilité du produit injecté, donc sa durée d'action, peut être modulée par la réticulation plus ou moins importante du gel.

## Revendications

1. Composition d'un médicament injectable et d'un gel dans le but de concentrer le produit sur place pour renforcer son action et/ou diminuer sa toxicité générale.

2. Composition selon la revendication 1, contenant un gel d'origine animale ou végétale.

3. Composition selon la revendication 1 ou 2, présentant plusieurs degrés de réticulation pour obtenir une résorbabilité plus ou moins prolongée.

4. Composition selon l'une quelconque des revendications 1 à 3, présentant une viscosité choisie parmi les ordres de grandeurs suivants : 5,27 PA/s, 8,3 PA/s, 17,5 PA/s.

5. Composition selon l'une quelconque des revendications 1 à 4, comportant un produit d'anesthésie dentaire.

6. Composition selon l'une quelconque des revendications 1 à 4, comportant tout autre produit actif et ses adjuvants.

7. Composition selon l'une quelconque des revendications 1 à 6, comportant une faible quantité de vasoactif.

8. Composition selon l'une quelconque des revendications 1 à 6, comportant au moins un conservateur et/ou au moins un antioxydant.
